(19) Europäisches Patentamt
European Patent Office
Office européen des brevets

(11) Veröffentlichungsnummer : **0 458 950 B1**

# (12) EUROPÄISCHE PATENTSCHRIFT

(45) Veröffentlichungstag der Patentschrift :
**15.09.93 Patentblatt 93/37**

(51) Int. Cl.$^5$ : **A61K 37/54,** A61K 47/00, A61K 47/06

(21) Anmeldenummer : **91901736.8**

(22) Anmeldetag : **19.12.90**

(86) Internationale Anmeldenummer :
**PCT/EP90/02250**

(87) Internationale Veröffentlichungsnummer :
**WO 91/08765 27.06.91 Gazette 91/14**

## (54) K2P PRO-STABILISIERUNG.

(30) Priorität : **20.12.89 DE 3942141**

(43) Veröffentlichungstag der Anmeldung :
**04.12.91 Patentblatt 91/49**

(45) Bekanntmachung des Hinweises auf die Patenterteilung :
**15.09.93 Patentblatt 93/37**

(84) Benannte Vertragsstaaten :
**AT BE CH DE DK ES FR GB GR IT LI LU NL SE**

(56) Entgegenhaltungen :
**EP-A- 0 211 592**
**EP-A- 0 228 862**
**EP-A- 0 297 294**
**WO-A-90/01333**
**WO-A-90/08557**
**Biochemistry, vol. 28, 1989, Am. Chemical Soc., R.F. Kelley et al., pp. 4047-4054**

(73) Patentinhaber : **BOEHRINGER MANNHEIM GMBH**
**D-68298 Mannheim (DE)**

(72) Erfinder : **KOHNERT, Ulrich, Dr.rer.nat.**
**Heubachweg 6**
**W-8121 Habach (DE)**
Erfinder : **RUDOLPH, Rainer, Dr.rer.nat.**
**Färbergasse 19**
**W-8120 Weilheim (DE)**

(74) Vertreter : **Prechtel, Jörg, Dipl.-Phys. Dr. et al**
**Patentanwälte H. Weickmann, Dr. K. Fincke F.A. Weickmann, B. Huber Dr. H. Liska, Dr. J. Prechtel Kopernikusstrasse 9 Postfach 86 08 20**
**D-81635 München (DE)**

**EP 0 458 950 B1**

Anmerkung : Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Der menschliche Gewebe-Plasminogen-Aktivator (t-PA) besitzt eine große therapeutische Bedeutung bei der Auflösung von Blutgerinnseln, z.B. bei Herzinfarkten. t-PA bewirkt die Auflösung von Blutgerinnseln durch die Aktivierung von Plasminogen zu Plasmin. Plasmin wiederum löst Fibrin, die Hauptkomponente der Proteinmatrix von geronnenem Blut.

Natürlicher t-PA ist aus mehreren funktionellen Domänen F, E, K1, K2 und P zusammengesetzt. Die Domäne P beinhaltet das proteolytisch aktive Zentrum, das die Spaltung von Plasminogen zu Plasmin bewirkt. Die gentechnologische Herstellung von t-PA oder verschiedenen t-PA-Muteinen, bei denen einige der Domänen F, E, K1 und K2 deletiert sind, in eukaryontischen und prokaryontischen Zellen ist bereits bekannt. Dabei werden t-PA-Derivate aus Prokaryonten im Gegensatz zu natürlichem t-PA in nicht glykosylierter Form synthetisiert.

Weiterhin ist bekannt, daß der Zuckeranteil einen erheblichen Einfluß auf die Löslichkeit und Aggregation von Proteinen hat (J. Biol. Chem. 263 (1988), 8832-8837). Es wurde nun festgestellt, daß ein nicht glykosyliertes t-PA-Mutein mit der Domänen-Zusammensetzung K2P eine wesentlich schlechtere Löslichkeit besitzt als etwa glykosylierte t-PA-Derivate. Diese nicht glykosylierte t-PA-Variante löst sich nur in geringem Ausmaß in den üblicherweise zur Solubilisierung von Proteinen verwendeten Puffern, wie z.B. 50 mmol/l Na-Citrat pH 6, 50 mmol/l Phosphat-Puffer oder physiologischer NaCl-Lösung . Für den Einsatz als therapeutischer Wirkstoff sollte jedoch das nicht glykosylierte t-PA-Derivat K2P pro mit einer deutlich höheren enzymatischen Aktivität von mindestens 1,4 MU/ml, vorzugsweise 1,4 MU/ml bis 10 MU/ml vorliegen.

Aus der EP-A-0 217 379 ist bekannt, die Löslichkeit von t-PA aus Prokaryonten (t-PA pro) durch neutrale oder leicht alkalische Arginin-Formulierungen zu erhöhen. Ein Nachteil dieses Verfahrens ist jedoch, daß gute Löslichkeiten von t-PA pro nur mit sehr hohen Argininkonzentrationen erreicht werden können. Außerdem ist die Stabilität des hochkonzentrierten t-PA-Derivats K2P pro unter neutralen oder leicht alkalischen Bedingungen gering.

Aufgabe der Erfindung ist es demnach, Formulierungen zu entwickeln, die das nicht glykosylierte t-PA-Derivat K2P pro mit einer enzymatischen Aktivität von mindestens 1,4 MU/ml enthalten, wobei die Stabilität des t-PA-Derivats über einen längeren Zeitraum hinweg erhalten bleiben soll.

Die erfindungsgemäße Aufgabe wird gelöst durch ein pharmazeutisches Präparat eines nicht glykosylierten t-PA-Derivats welches aus der Kringel 2-Domäne und der Proteasedomäne besteht und mit einer der Aminosäuren 174 - 180 beginnt und mit der Aminosäure 527 endet und außerdem die Aminosäuren -3 (Gly) bis +5 (Ile) ganz oder teilweise enthalten kann (K2P pro) mit einer enzymatischen Aktivität von mindestens 1,4 MU/ml und einem pH-Wert von 4,5 bis 6,5, wobei diese Zusammensetzung Citrat und mindestens eine Verbindung aus der aus

a) Ascorbinsäure,

b) EDTA,

c) Aminoverbindungen der Formel

$$R^1R^2N - R - X$$

wobei X = $SO_3H$, CH $(NH_2)$-$CO_2H$, $CO_2H$, H, $NH_2$ oder OH ist, R = $C_1$-$C_9$-Alkylen, vorzugsweise $C_4$-$C_7$-Alkylen, $C_3$-$C_6$-Cycloalkylen oder Benzyliden ist und $R^1$ und $R^2$ voneinander unabhängig H oder $C_1$-$C_3$-Alkyl sind,

d) Guanidin-analoge Verbindungen der Formel

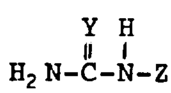

wobei

Y = $H_2N^+$ oder 0 ist,

Z = H oder $(CH_2)_m$ V, $(CH_2)_mCH(NH_2)$-$CO_2H$, $CH(CO_2H)$-$(CH_2)_mCO_2H$ ist,

V = $NH_2$ oder $CO_2H$ und

m = 1 bis 4 ist

e) mit einer oder mehreren Hydroxyl-, Keto- oder/und weiteren Carboxylgruppen substituierten Carbonsäuren,

f) Dimethylbiguanid,

g) Pyrimidinnukleosiden und Pyridinnukleotiden,

h) Trehalose, Glucosamin, N-Methylglucamin bestehenden Gruppe enthält.

Unter K2P pro gemäß vorliegender Erfindung versteht man ein t-PA-Derivat, welches aus der Kringel 2- und der Proteasedomäne besteht und damit bei einer der Aminosäuren 174-180 beginnt und mit der Aminosäure 527 endet. K2P pro kann zusätzlich noch teilweise oder ganz die Aminosäuren -3 (Gly) bis +5 (Ile) enthalten. Dabei ist ein Protein bevorzugt, das bei Aminosäure 176 beginnt und ggf. aus dem Bereich -3 bis +5 noch die Aminosäuren Ser, Tyr, Gln vorgeschaltet enthält. Diese Bezeichnung folgt der in T. J. R. Harris, Protein Engineering, Band 1 (1987) 449-458 für t-PA angegebenen Nomenklatur. Die Herstellung derartiger t-PA-Derivate K2P pro ist in der EP-A 0 382 174 beschrieben. Die enzymatische Aktivität für K2P pro ist als Standardeinheit U gemäß der Definition der WHO für t-PA angegeben. Die Bestimmung der Aktivität erfolgt nach H. Lill, ZGIMAL 42 (1987), 478-486.

Für die Solubilisierung von K2P pro hat sich ein Citratpuffer als besonders geeignet erwiesen. Die Citratkonzentration z.B. soll mindestens 5 mmol/l, vorzugsweise 5 bis 100 mmol/l, besonders bevorzugt 50 mmol/l betragen. Der pH-Wert wird nach Basizität der zugesetzten Verbindung vorzugsweise mit HCl oder einer Base wie z.B. NaOH oder KOH eingestellt.

Überraschenderweise wurde festgestellt, daß die Löslichkeit von nicht glykosyliertem K2P pro in anderen Puffersystemen, z.B. Phosphatpuffer, bei gleicher Ionenstärke und gleichem pH-Wert wesentlich geringer ist. Es hat sich als geeignet erwiesen, den pH-Wert von alkalischen Citrat-Lösungen mit HCl einzustellen, d.h. daß die Zusammensetzung zusätzlich noch Chloridionen enthält. In Gegenwart von Chloridionen sind nämlich überraschenderweise hochkonzentrierte Lösungen von nicht glykosyliertem K2P pro wesentlich stabiler als z.B. in Gegenwart von Phosphationen. Der pH-Wert von sauren Citrat-Lösungen wird üblicherweise mit NaOH eingestellt.

Geeignet für ein erfindungsgemäßes pharmazeutisches Präparat ist ein pH-Wert zwischen 4,5 und 6,5, bevorzugt ist ein pH-Wert von 5 bis 6. Überraschenderweise nimmt bei den üblicherweise für natives t-PA verwendeten Formulierungen mit einem pH-Wert von > 7 die Stabilität des nicht glykosylierten t-PA-Derivats K2P pro in der Lösung erheblich ab. So ist die einkettige Form von K2P pro bei pH 7,2 und pH 8 in Arginin-gepufferten Lösungen nur wenige Tage bei Raumtemperatur oder höheren Temperaturen stabil.

Ein erfindungsgemäßes pharmazeutisches Präparat enthält Ascorbinsäure, vorzugsweise von 0,1 bis 1 mol/l, besonders bevorzugt von 0,2 bis 0,3 mol/l.

Die Konzentration von EDTA sollte vorzugsweise 1 bis 200 mol/l, besonders bevorzugt 10 bis 100 mmol/l betragen.

Vorzugsweise werden für eine erfindungsgemäße Zusammensetzung als Aminoverbindungen Taurin, ε-Aminocapronsäure, Tranexamsäure, Lysin, Ornithin, δ-Aminovaleriansäure, p-Aminomethylbenzoesäure, 8-Aminooctansäure oder/und 7-Aminoheptansäure verwendet. Besonders bevorzugt ist die Verwendung von ε-Aminocapronsäure, p-Aminomethylbenzoesäure, 7-Aminoheptansäure, 8-Aminooctansäure, Tranexansäure oder/und Lysin. Die bevorzugten Konzentrationen betragen 0,5 bis 20 mmol/l, besonders bevorzugt 1 bis 10 mmol/l.

Ebenso geeignet sind 4-Aminobutanol-1, 5-Aminopentanol-1, 6-Aminohexanol-1, 1,9-Diaminononan, 1,8-Diaminooctan, 1,7-Diaminoheptan, 1,6-Diaminohexan, 1,5-Diaminopentan, 1,4-Diaminobutan oder/und 1,3-Diaminopropan. Die Konzentrationen der für ein erfindungsgemäßes Präparat geeigneten α,ω-Diamine und α,ω-Aminoalkohole betragen vorzugsweise 10 bis 100 mmol/l.

Taurin und analoge Verbindungen werden vorzugsweise mit 0,1 bis 0,5 mol/l, besonders bevorzugt mit 0,1 bis 0,3 mol/l verwendet.

Weiterhin werden für eine erfindungsgemäße Zusammensetzung als Guanidin-analoge Verbindungen vorzugsweise Harnstoff, Guanidinobuttersäure oder/und Arginin verwendet. Die Konzentration von Harnstoff beträgt vorzugsweise 0,1 bis 4 mol/l, besonders bevorzugt 0,5 bis 2 mol/l. Für andere Guanidin-analoge Verbindungen beträgt die Konzentration vorzugsweise 10 bis 200 mmol/l, besonders bevorzugt 50 bis 100 mmol/l.

Als Carbonsäuren, die mit Hydroxy-, Keto- oder/und weiteren Carboxylgruppen substituiert sind, sind z.B. Apfelsäure, Milchsäure, Fumarsäure oder/und 2-Oxoglutarsäure geeignet. Ihre Konzentration beträgt vorzugsweise 0,001 bis 1 mol/l, besonders bevorzugt 0,01 bis 0,5 mol/l.

Dimethylbiguanid wird bevorzugt in Konzentrationen von 50 bis 400 mmol/l, besonders bevorzugt mit 100 bis 300 mmol/l eingesetzt.

Als Pyrimidinnukleosid oder Pyrimidinnukleotid sind z.B. Thymidin, Cytosin und Uridin bzw. die entsprechenden Nukleotide geeignet. Diese Substanzen werden bevorzugt in Konzentrationen von 1 bis 300 mmol/l, besonders bevorzugt von 10 bis 300 mmol/l eingesetzt.

Trehalose, Glucosamin und N-Methylglucamin werden bevorzugt in Konzentrationen von 1 bis 500 mmol/l, besonders bevorzugt mit 10 bis 300 mmol/l eingesetzt.

Weiterhin ein Gegenstand der Erfindung ist eine erfindungsgemäße Zusammensetzung, die zusätzlich eine oder mehrere α-Aminocarbonsäuren, insbesondere Histidin enthält.

Im folgenden ist eine Reihe besonders bevorzugter Präparate gemäß vorliegender Erfindung aufgeführt.

Eine Formulierung enthält 50 mmol/l Na-Citrat/HCl, pH 6, 2 mol/l Harnstoff. Eine weitere Formulierung enthält 50 mmol/l Na-Citrat, pH 6 und 0,5 mol/l bis 1 mol/l Guanidin. Wiederum eine weitere Formulierung enthält 50 mmol/l Na-Citrat, pH 6 und 0,3 mol/l Taurin. Wiederum eine weitere Formulierung enthält 50 mmol/l Na-Citrat, pH 6 und 0,2 mol/l bis 0,3 mol/l Ascorbinsäure. Wiederum eine weitere Formulierung enthält 50 mmol/l Na-Citrat/ HCl, pH 6 und 300 mmol/l Dimethylbiguanid. Eine weitere Formulierung enthält 50 mmol/l NaCitrat/HCl, pH 6 und 10 bis 300 mmol/l Thymidin, Uridin oder Cytosin bzw. 10 bis 100 mmol/l eines der obengenannten $\alpha,\omega$-Diamine oder einen der obengenannten $\alpha,\omega$-Aminoalkohole. Eine weitere Formulierung enthält 50 mmol/l NaCitrat/HCl, pH 6 und 10 bis 300 mmol/l Trehalose, Glucosamin oder N-Methylglucamin.

Wiederum eine besonders bevorzugte erfindungsgemäße Formulierung enthält 50 mmol/l Na-Citrat/HCl, pH 6 und 1 mmol/l bis 10 mmol/l $\epsilon$-Aminocapronsäure, $\delta$-Aminovaleriansäure, 7-Aminoheptansäure, 8-Aminooctansäure, p-Aminomethylbenzoesäure, L-Lysin, Ornithin oder Tranexamsäure. Diese Substanzen bewirken überraschenderweise bereits in geringem molaren Überschuß (10- bis 40fach) eine hervorragende Löslichkeit von K2P pro.

Weiterhin bevorzugt sind auch Formulierungen, die 50 mmol/l Na-Citrat/HCl, pH 6 und eine Guanidin-analoge Verbindung, insbesondere Arginin und Guanidinobuttersäure in einer Konzentration von 50 bis 100 mmol/l enthalten. Eine weitere Formulierung enthält 50 mmol/l NaCitrat, pH 6 und 10 bis 500 mmol/l Apfelsäure, Milchsäure, Fumarsäure oder 2-Oxoglutarsäure. Wiederum eine weitere Formulierung enthält 50 mmol/l Na-Citrat, pH 6 und 10 bis 100 mmol/l EDTA.

Wie aus den Beispielen ersichtlich ist, bewirken auch Kombinationen aus mehreren der obengenannten Verbindungen mit Citrat eine sehr gute Löslichkeit von K2P pro. Geeignet sind z.B. Kombinationen von Lysin mit Arginin, Ornithin, Glucosamin oder/und Thymidin oder von EDTA mit $\epsilon$-Aminocapronsäure, Lysin, Arginin, Glucosamin oder/und Thymidin. Ebenso geeignet sind jedoch auch andere Kombinationen von mindestens 2 der obengenannten Verbindungen mit Citrat.

Ein Gegenstand der Erfindung ist schließlich auch ein Arzneimittel auf Basis von K2P pro als Wirkstoff in Lösung oder als Lyophilisat mit den angegebenen Substanzen und gegebenenfalls noch weiteren pharmazeutisch verträglichen Zusatz-, Hilfs-, Träger- und Füllstoffen.

Die erfindungsgemäßen pharmazeutischen Präparate kommen vorzugsweise als Injektions- und Infusionslösungen zum Einsatz. Dies kann dadurch geschehen, daß eine bereits spritzfertige Lösung zur Verfügung gestellt wird, die die erfindungsgemäße Zusammensetzung besitzt. Es ist jedoch auch möglich, die pharmazeutischen Präparate in Form von Lyophilisaten zur Verfügung zu stellen. Diese werden dann mit an sich bekannten, zu Injektionszwecken geeigneten Mitteln oder Lösungen rekonstituiert. Als Injektionsmedium kommt vorzugsweise Wasser zur Anwendung, welches die bei Injektionslösungen üblichen Zusätze wie Stabilisierungsmittel, Lösungsvermittler, Puffer und isotonische Zusätze, beispielsweise eine physiologische NaCl-Konzentration, enthält. Derartige Zusätze sind beispielsweise Mannit, Tartrat- oder Citrat-Puffer, Ethanol, Komplexbildner wie z.B. Ethylendiamintetraessigsäure und deren nicht-toxischen Salze, sowie hochmolekulare Polymere, wie flüssiges Polyethylenoxid zur Viskositätsregulierung. Flüssige Trägerstoffe für Injektionslösungen müssen steril sein und werden vorzugsweise in Ampullen abgefüllt.

Schließlich beinhaltet die vorliegende Erfindung auch die Verwendung von K2P pro zur Herstellung von erfindungsgemäßen pharmazeutischen Präparaten.

Die folgenden Beispiele sollen konkrete Ausführungsformen der Erfindung weiter erläutern.

**Beispiel 1**

Einfluß von Harnstoff auf die Löslichkeit eines nicht glykosylierten t-PA-Muteins mit der Domänen-Zusammensetzung K2P

In diesem Beispiel wird der Einfluß von Harnstoff auf die Löslichkeit von K2P pro (Herstellung nach EP-A 0 382 174) in Citrat-gepufferten Lösungen bei pH 6.0 beschrieben. Wie aus Tabelle 1 zu entnehmen ist, ist K2P pro in 50 mmol/l Citrat-Puffer bei pH 6.0 nur bedingt löslich. Durch Zusatz von Harnstoff kann die Löslichkeit erheblich verbessert werden. Das Optimum liegt dabei etwa bei 2 mol/l Harnstoff.

Durchführung

170 ml gereinigtes K2P pro (gelöst in 0,5 mol/l Arginin/$H_3PO_4$, pH 7,2) werden durch Ultrafiltration über eine Amicon YM 10-Membran konzentriert. Jeweils 1 ml des Konzentrats (Aktivität 5,8 MU/ml) wird gegen die in Tabelle 1 aufgeführten Puffer dialysiert. Nach Zentrifugation der Proben wird im klaren Überstand die enzymatische Aktivität gemessen.

Die enzymatische Aktivität ist als Volumenaktivität in MU/ml und als Gesamtaktivität in MU angegeben.

Die Messung der K2P-Aktivität kann dabei auf übliche Weise durch Spaltung eines chromogenen Substrats

bestimmt werden (H. Lill, ZGIMAL 42 (1987), 478-486). Die Einheit U ist eine Einheit der Aktivität für t-PA nach Definition der WHO, National Institute for Biological Standards and Control.

Tabelle 1

| Puffer | Aktivität | |
|---|---|---|
| | MU/ml | MU |
| 50 mmol/l NaCitrat/HCl, pH 6.0<br>8 mol/l Harnstoff | 1.03 | 1.24 |
| 50 mmol/l NaCitrat/HCl, pH 6.0<br>6 mol/l Harnstoff | 2.76 | 3.59 |
| 50 mmol/l NaCitrat/HCl, pH 6.0<br>4 mol/l Harnstoff | 3.46 | 4.67 |
| 50 mmol/l NaCitrat/HCl, pH 6.0<br>2 mol/l Harnstoff | 4.20 | 5.67 |
| 50 mmol/l NaCitrat/HCl, pH 6.0<br>1 mol/l Harnstoff | 2.62 | 3.27 |
| 50 mmol/l NaCitrat/HCl, pH 6.0 | 0.32 | 0.93 |

**Beispiel 2**

Einfluß von verschiedenen Substanzen auf die Löslichkeit von K2P pro

In diesem Beispiel wird der Einfluß von verschiedenen Substanzen auf die Löslichkeit von K2P pro in Citrat-gepufferten Lösungen bei pH 6 beschrieben. Hervorragende Löslichkeiten (> 2 MU/ml) wurden mit Taurin, Ascorbinsäure, Lysin, EACA, Tranexamsäure, Dimethylbiguanid, Glucosamin, Trehalose, N-Methylglucamin, Uridin, Cytidin, p-Aminomethylbenzoesäure, Fumarsäure und Oxoglutarsäure erzielt. Weiterhin ist ersichtlich, daß der Citratpuffer bei gleicher molarer Konzentration eine bessere Löslichkeit des t-PA-Derivats als ein $NH_4HCO_3$-, Tris- oder Phosphatpuffer bewirkt.

Durchführung:

siehe Beispiel 1

Konzentrat:

Aktivität: 5.8 MU/ml

T a b e l l e 2

| Puffer | Aktivität | |
|---|---|---|
| | MU/ml | MU |
| 50 mmol/l NaCitrat/HCl, pH 6.0<br>50 mmol/l Ornithin | 1.92 | 2.11 |
| 50 mmol/l NaCitrat/NaOH, pH 6.0<br>0.3 mol/l Taurin | 2.67 | 4.00 |
| 50 mmol/l NaCitrat/NaOH, pH 6.0<br>0.3 mol/l Ascorbinsäure | 4.00 | 4.20 |
| 50 mmol/l NaCitrat/HCl, pH 6.0<br>10 mmol/l EACA | 3.88 | 5.80 |
| 50 mmol/l NaCitrat/HCl, pH 6.0<br>10 mmol/l L-Lysin | 2.46 | 3.32 |
| 50 mmol/l NaCitrat/HCl, pH 6.0<br>10 mmol/l Tranexamsäure | 5.54 | 7.36 |
| 50 mmol/l NaCitrat/HCl, pH 6.0<br>0.3 mol/l Dimethylbi-guanid | 2.96 | 3.40 |
| 50 mmol/l Tris/HCl, pH 7.2 | 0.04 | 0.06 |
| 50 mmol/l $NH_4HCO_3$ | 0.12 | 0.19 |
| 50 mmol/l $Na_2HPO_4/H_3PO_4$, pH 7.2 | 0.15 | 0.23 |

| Puffer | Aktivität | |
|---|---|---|
| | MU/ml | MU |
| 50 mmol/l Na-Citrat/HCl, pH 6.0 | 0.32 | 0.93 |
| 50 mmol/l NaCitrat/HCl, pH 6<br>0.3 mol/l Glucosamin | 2.02 | 2.02 |
| 50 mmol/l NaCitrat/HCl, pH 6<br>0.3 mol/l Trehalose | 3.52 | 3.17 |
| 50 mmol/l NaCitrat/HCl, pH 6<br>0.1 mol/l Thymidin | 1.57 | 1.88 |
| 50 mmol/l NaCitrat/HCl, pH 6<br>0.3 mol/l Uridin | 6.32 | 7.58 |
| 50 mmol/l NaCitrat/HCl, pH 6<br>30 mmol/l Cytosin | 3.8 | 4.94 |
| 50 mmol/l NaCitrat/HCl, pH 6<br>10 mmol/l p-Aminomethyl-benzoesäure | 3.46 | 5.03 |
| 50 mmol/l NaCitrat/NaOH, pH 6<br>0.3 mol/l Apfelsäure | 1.53 | 1.68 |
| 50 mmol/l NaCitrat/NaOH, pH 6<br>0.3 mol/l Milchsäure | 1.59 | 1.98 |
| 50 mmol/l NaCitrat/NaOH, pH 6<br>0.3 mol/l Fumarsäure | 4.32 | 5.16 |
| 50 mmol/l NaCitrat/NaOH, pH 6<br>0.3 mol/l 2-Oxoglutarsäure | 4.24 | 4.66 |
| 50 mmol/l NaCitrat/HCl, pH 6<br>0.3 mol/l N-Methylglucamin | 3.36 | 3.70 |

**Beispiel 3**

Einfluß von ∊-Aminocapronsäure (EACA) auf die Löslichkeit von K2P pro

Durchführung:

siehe Beispiel 1

Konzentrat:

Aktivität: 6.3 MU/ml

Tabelle 3

| Puffer | Aktivität | |
|---|---|---|
| | MU/ml | MU |
| 50 mmol/l NaCitrat/HCl, pH 6.0 | 0.34 | 0.48 |
| 50 mmol/l NaCitrat/HCl, pH 6.0<br>10 mmol/l EACA | 3.81 | 4.57 |
| 50 mmol/l NaCitrat/HCl, pH 6.0<br>5 mmol/l EACA | 3.52 | 4.86 |
| 50 mmol/l NaCitrat/HCl, pH 6.0<br>1 mmol/l EACA | 2.70 | 3.83 |
| 50 mmol/l NaCitrat/HCl, pH 6.0<br>0.1 mmol/l EACA | 0.58 | 0.75 |

Tabelle 3 zeigt, daß EACA schon in einer Konzentration von 1 - 10 mmol/l (10 - 40facher molarer Überschuß im Vergleich zu K2P pro) eine erhebliche Verbesserung der Löslichkeit in Citrat-gepufferten Lösungen bei pH 6.0 bewirkt.

**Beispiel 4**

Einfluß von Ascorbinsäure auf die Löslichkeit von K2P pro

Durchführung:

siehe Beispiel 1

Konzentrat:

Aktivität: 6.3 MU/ml

Tabelle 4

| Puffer | Aktivität | |
|---|---|---|
| | MU/ml | MU |
| 50 mmol/l NaCitrat/HCl, pH 6.0 | 0.34 | 0.48 |
| 50 mmol/l NaCitrat/NaOH, pH 6.0<br>0.3 mol/l Ascorbinsäure | 4.04 | 4.24 |
| 50 mmol/l NaCitrat/NaOH, pH 6.0<br>0.2 mol/l Ascorbinsäure | 2.40 | 2.93 |

Die Ergebnisse von Tabelle 4 zeigen, daß in Citrat-gepufferten Lösungen die Löslichkeit von K2P pro durch Zusatz von Ascorbinsäure weiter erhöht wird.

**Beispiel 5**

pH-Abhängigkeit der Stabilität von K2P pro in Argininhaltigen Lösungen

Gereinigtes K2P pro wird gegen die in Tabelle 5 aufgeführten 0.5 mol/l Arg/$H_3PO_4$-Puffer dialysiert und portioniert bei - 20, 25 und 37°C gelagert. Nach 3, 7, 14 und 21 Tagen wird jeweils 1 Probe auf Aktivität und Stimulierbarkeit durch Zugabe von Fibrin getestet und SDS-elektrophoretisch analysiert (Ausgangswerte: Aktivität: 1,3 MU/ml, Stimulierbarkeit: 28). Die in Tabelle 5 zusammengefaßten Daten zeigen, daß die bei pH 8 und pH 7.2 gelagerten Proben nach 3 bzw. 7 Tagen Lagerung bei 37 °C eine deutliche Erhöhung der Aktivität unter gleichzeitiger Abnahme der Stimulierbarkeit aufweisen. Bei 25 °C kommt es, allerdings zeitlich verzögert, ebenfalls zu einem Anstieg der Aktivität und Abnahme der Stimulierbarkeit. Nach 14 bis 21 Tagen wird dann auch eine Verringerung der Aktivität beobachtet.

Tabelle 5

| Zeit | Aktivität (MU/ml) /Stimulierbarkeit -20 °C | 25 °C | 37 °C |
|---|---|---|---|
| pH 8.0 | | | |
| 3 Tage | 1.22/36 | 1.47/24 | 1.45/15 |
| 7 Tage | 1.23/44 | 1.87/15 | 2.38/13 |
| 14 Tage | 1.37/33 | 2.08/9 | 1.1/10 |
| 21 Tage | 1.37/- | 2.14/10 | 1.0/7 |
| pH 7.2 | | | |
| 3 Tage | 1.12/39 | 1.5/27 | 1.7/20 |
| 7 Tage | 1.16/45 | 1.7/21 | 2.1/13 |
| 14 Tage | 1.38/32 | 1.7/9 | 1.9/9 |
| 21 Tage | 1.22/25 | 1.7/11 | 1.52/8 |
| pH 6.0 | | | |
| 3 Tage | 0.93/34 | 1.43/31 | 1.54/34 |
| 7 Tage | 1.05/46 | 1.27/42 | 1.58/36 |
| 14 Tage | 1.23/39 | 1.04/20 | 1.45/14 |
| 21 Tage | 1.43/26 | 0.95/16 | 1.00/8 |
| pH 5.5 | | | |
| 3 Tage | 0.85/32 | 1.47/40 | 1.08/39 |
| 7 Tage | 1.4/47 | 1.53/68 | 1.54/48 |
| 14 Tage | 1.2/32 | 1.3/26 | 1.3/24 |
| 21 Tage | 1.36/26 | 0.95/25 | 0.95/21 |

Parallel zu diesen Veränderungen wird eine Abnahme des Molekulargewichts von K2P pro (Elektrophorese mit SDS-PAGE) beobachtet.

**Beispiel 6**

Einfluß von Chlorid- und Phosphat-Ionen auf die Stabilität von K2P pro in Arginin (Arg)-haltigen Lösungen

Gereinigtes K2P pro wird gegen die unten aufgeführten Arginin-haltigen Puffer dialysiert und portioniert bei -20, 25 und 37 °C gelagert. Nach 2, 7 und 14 Tagen wird jeweils 1 Probe SDS-elektrophoretisch analysiert. Es zeigt sich, daß in Gegenwart von Chlorid-Ionen die einkettige Form wesentlich stabiler ist als in Gegenwart von Phosphat-Ionen. Während in Chloridionen-haltigen Lösungen nach 7 Tagen Lagerung bei 37 °C bei pH 7.2 und 8 erst 10 % bis 20 % der Probe gespalten sind, beträgt in den Phosphat-gepufferten Lösungen der Anteil des gespaltenen Materials 60 % bis 90 %.

Puffer:

0.5 mol/l Arg/$H_3PO_4$, pH 8.0
0.5 mol/l Arg/HCl, pH 8.0
0.5 mol/l Arg/$H_3PO_4$, pH 7.2
0.5 mol/l Arg/HCl, pH 7.2

**Beispiel 7**

Einfluß von ε-Aminocapronsäure auf die Löslichkeit von K2P pro in Citrat- und Phosphat-gepufferten Lösungen

Gereinigtes K2P pro wird durch Ultrafiltration auf 4.2 MU/ml konzentriert und gegen die in Tabelle 8 angegebenen Puffer dialysiert und zentrifugiert. Nach Zentrifugation der Proben wird im klaren Überstand die Aktivität gemessen.

Tabelle 6

| Puffer | Aktivität | |
|---|---|---|
| | MU/ml | MU |
| 50 mmol/l NaCitrat/HCl, pH 6 | 0.32 | 0.93 |
| 50 mmol/l $Na_2HPO_4/H_3PO_4$, pH 6 | 0.07 | 0.10 |
| 50 mmol/l NaCitrat/HCl, pH 6<br>2 mmol/l EACA | 3.30 | 4.60 |
| 50 mmol/l $Na_2HPO_4/H_3PO_4$, pH 6<br>2 mmol/l EACA | 0.90 | 1.39 |
| 50 mmol/l NaCitrat/HCl, pH 6<br>2 mmol/l EACA<br>0.15 mol/l NaCl | 2.43 | 3.52 |
| 50 mmol/l $Na_2HPO_4/H_3PO_4$, pH 6<br>2 mmol/l EACA<br>0,15 mol/l NaCl | 1.08 | 1.57 |

Tabelle 6 zeigt, daß die Verbesserung der Löslichkeit durch EACA in Citrat-gepufferten Lösungen wesentlich besser ist als in Phosphat-Puffern.

**Beispiel 8**

Einfluß von Tranexamsäure (TES) auf die Löslichkeit von K2P pro

Durchführung:

siehe Beispiel 1

Konzentrat:

Aktivität: 4.2 MU/ml

Tabelle 7

| Puffer | Aktivität MU/ml | MU |
|---|---|---|
| 50 mmol/l NaCitrat/HCl, pH 6<br>10 mmol/l TES | 2.66 | 3.60 |
| 50 mmol/l NaCitrat/HCl, pH 6<br>5 mmol/l TES | 2.54 | 3.30 |
| 50 mmol/l NaCitrat/HCl, pH 6<br>1 mmol/l TES | 2.35 | 3.29 |

Die Ergebnisse zeigen, daß mit Tranexamsäure (TES)[1)] eine ähnliche Löslichkeit von K2P pro zu erzielen ist wie mit EACA.

1) trans-4-Aminomethylcyclohexancarbonsäure

**Beispiel 9**

Einfluß von ω-Aminocarbonsäuren auf die Löslichkeit von K2P pro

Durchführung:

siehe Beispiel 1

Konzentrat:

Aktivität: 4.2 MU/ml

Tabelle 8

| Puffer | Aktivität MU/ml | MU |
|---|---|---|
| 50 mmol/l NaCitrat/HCl, pH 6 | 0.32 | 0.93 |
| 50 mmol/l NaCitrat/HCl, pH 6<br>10 mmol/l 8-Aminooctansäure | 3.12 | 4.05 |
| 50 mmol/l NaCitrat/HCl, pH 6<br>10 mmol/l 7-Aminoheptansäure | 3.54 | 4.42 |
| 50 mmol/l NaCitrat/HCl, pH 6<br>10 mmol/l EACA | 4.22 | 2.90 |
| 50 mmol/l NaCitrat/HCl, pH 6<br>10 mmol/l δ-Aminovaleriansäure | 1.83 | 2.47 |

**Beispiel 10**

Einfluß von Guanidin-Analoga auf die Löslichkeit von K2P pro

Durchführung:

siehe Beispiel 1

Konzentrat:

Aktivität: 4.2 MU/ml

Tabelle 9

| Puffer | Aktivität | |
|---|---|---|
| | MU/ml | MU |
| 50 mmol/l NaCitrat/HCl, pH 6<br>50 mmol/l Arginin | 1.42 | 2.06 |
| 50 mmol/l NaCitrat/HCl, pH 6<br>50 mmol/l Guanidinobuttersäure | 2.21 | 3.08 |

Die Ergebnisse zeigen, daß mit einer Guanidinogruppe die Löslichkeit von K2P pro deutlich verbessert wird.

**Beispiel 11**

Einfluß von EDTA auf das Löslichkeitsverhalten von K2P pro

Durchführung:

siehe Beispiel 1

Konzentrat:

Aktivität: 4.2 MU/ml

Tabelle 10

| Puffer | Aktivität | |
|---|---|---|
| | MU/ml | MU |
| 10 mmol/l EDTA/NaOH, pH 6 | 0.02 | 0.03 |
| 0,3 mol/l EDTA/NaOH, pH 6 | 1.95 | 1.95 |
| 50 mmol/l NaCitrat/NaOH, pH 6<br>50 mmol/l EDTA | 1.80 | 2.25 |
| 50 mmol/l NaCitrat/NaOH, pH 6<br>100 mmol/l EDTA | 3.36 | 4.03 |
| 50 mmol/l NaCitrat/HCl, pH 6 | 0.32 | 0.93 |

Tabelle 10 zeigt, daß die Kombination von EDTA mit Citrat einen mehr als additiven Effekt auf die Löslichkeit von K2P pro hat.

**Beispiel 12**

Einfluß von Aminosäuren alleine oder in Kombination auf die Löslichkeit von K2P pro

Durchführung:

siehe Beispiel 1

Konzentrat:

Aktivität: 4.2 MU/ml

Tabelle 11

| Puffer | Aktivität MU/ml | Aktivität MU |
|---|---|---|
| 50 mmol/l NaCitrat/HCl, pH 6<br>50 mmol/l Arginin | 1.42 | 2.06 |
| 50 mmol/l Nacitrat/HCl, pH 6<br>10 mmol/l L-Lysin | 2.81 | 3.94 |
| 50 mmol/l NaCitrat/HCl, pH 6<br>1 mmol/l L-Lysin | 2.00 | 2.6 |
| 50 mmol/l NaCitrat/HCl, pH 6<br>10 mmol/l L-Lysin<br>10 mmol/l Arginin | 2.56 | 3.84 |
| 50 mmol/l NaCitrat/HCl, pH 6<br>10 mmol/l Lysin<br>50 mmol/l Arginin | 3.27 | 4.9 |
| 50 mmol/l NaCitrat/HCl, pH 6<br>10 mmol/l Lysin<br>10 mmol/l Arginin<br>10 mmol/l Ornithin | 3.18 | 4.13 |
| 50 mmol/l NaCitrat/HCl, pH 6<br>10 mmol/l Lysin<br>50 mmol/l Arginin<br>10 mmol/l Ornithin | 2.70 | 3.78 |
| 50 mmol/l NaCitrat/HCl, pH 6<br>10 mmol/l Lysin<br>50 mmol/l Arginin<br>50 mmol/l Ornithin | 3.34 | 4.30 |

**Beispiel 13**

Einfluß von ω-Aminoalkoholen auf die Löslichkeit von K2P pro

Durchführung:

siehe Beispiel 1

**Konzentrat:**

Aktivität: 4.9 MU/ml

Tabelle 12

| Puffer | Aktivität | |
|---|---|---|
| | MU/ml | MU |
| 50 mmol/l NaCitrat/HCl, pH 6<br>50 mmol/l 4-Aminobutanol-1 | 1.84 | 2.4 |
| 50 mmol/l NaCitrat/HCl, pH 6<br>50 mmol/l 5-Aminopentanol-1 | 2.54 | 3.55 |

**Beispiel 14**

Einfluß von verschiedenen Substanzen in Kombination auf die Löslichkeit von K2P pro

Durchführung:

siehe Beispiel 1

**Konzentrat:**

Aktivität: 5.5 MU/ml

Tabelle 13

| Puffer | Aktivität | |
|---|---|---|
| | MU/ml | MU |
| 50 mmol/l NaCitrat, pH 6,<br>50 mmol/l EDTA<br>1 mmol/l EACA<br>50 mmol/l Glucosamin | 4.24 | 5.08 ~~pH~~ |
| 50 mmol/l NaCitrat, pH 6<br>10 mmol/l EDTA<br>1 mmol/l EACA<br>10 mmol/l Glucosamin | 3.63 | 5.08 |
| 50 mmol/l NaCitrat, pH 6<br>10 mmol/l EDTA<br>1 mmol/l EACA<br>10 mmol/l Glucosamin<br>50 mmol/l Thymidin | 3.70 | 5.14 |
| 50 mmol/l NaCitrat, pH 6<br>50 mmol/l EDTA<br>1 mmol/l Lysin<br>50 mmol/l Glucosamin | 4.00 | 5.40 |
| 50 mmol/l NaCitrat, pH 6<br>50 mmol/l EDTA<br>1 mmol/l Lysin<br>10 mmol/l Glucosamin | 3.04 | 3.95 |
| 50 mmol/l NaCitrat, pH 6<br>10 mmol/l EDTA<br>1 mmol/l Lysin<br>10 mmol/l Glucosamin<br>10 mmol/l Thymidin | 3.12 | 4.52 |
| 50 mmol/l NaCitrat, pH 6<br>10 mmol/l EDTA<br>1 mmol/l Lysin<br>10 mmol/l Arginin<br>10 mmol/l Glucosamin<br>10 mmol/l Thymidin | 2.60 | 3.77 |

| Puffer | Aktivität | |
|---|---|---|
| | MU/ml | MU |
| 50 mmol/l NaCitrat, pH 6<br>10 mmol/l EDTA<br>1 mmol/l Lysin<br>25 mmol/l Arginin<br>10 mmol/l Glucosamin<br>10 mmol/l Thymidin | 2.94 | 4.12 |
| 50 mmol/l NaCitrat, pH 6<br>1 mmol/l Lysin<br>10 mmol/l Arginin<br>10 mmol/l Glucosamin<br>10 mmol/l Thymidin | 2.80 | 3.64 |
| 50 mmol/l NaCitrat, pH 6<br>10 mmol/l EDTA<br>1 mmol/l Lysin<br>25 mmol/l Arginin<br>10 mmol/l Glucosamin<br>25 mmol/l Thymidin | 3.20 | 4.32 |
| 50 mmol/l NaCitrat, pH 6<br>10 mmol/l EDTA<br>1 mmol/l Lysin<br>25 mmol/l Arginin<br>10 mmol/l Glucosamin<br>50 mmol/l Thymidin | 4.56 | 5.90 |
| 50 mmol/l NaCitrat, pH 6<br>1 mmol/l Lysin<br>25 mmol/l Arginin<br>10 mmol/l Glucosamin<br>50 mmol/l Thymidin | 3.24 | 4.54 |

| Puffer | Aktivität | |
|---|---|---|
| | MU/ml | MU |
| 50 mmol/l NaCitrat, pH 6<br>1 mmol/l Lysin<br>25 mmol/l Arginin<br>10 mmol/l Glucosamin<br>25 mmol/l Thymidin | 3.54 | 4.95 |
| 50 mmol/l NaCitrat, pH 6<br>1 mmol/l Lysin<br>25 mmol/l Arginin<br>50 mmol/l Glucosamin<br>25 mmol/l Thymidin | 3.60 | 4.68 |
| 50 mmol/l NaCitrat, pH 6<br>1 mmol/l Lysin<br>25 mmol/l Arginin<br>100 mmol/l Glucosamin<br>25 mmol/l Thymidin | 5.85 | 5.85 |

**Beispiel 15**

Einfluß von $\alpha,\omega$-Diaminen auf die Löslichkeit von K2P pro

Durchführung:

siehe Beispiel 1

**Konzentrat:**

Aktivität: 4.9 MU/ml

Tabelle 14

| Puffer | Aktivität | |
| --- | --- | --- |
| | MU/ml | MU |
| 50 mmol/l NaCitrat/HCl, pH 6<br>50 mmol/l 1,9-Diaminononan | 3.12 | 4.05 |
| 50 mmol/l NaCitrat/HCl, pH 6<br>50 mmol/l 1,8-Diaminooctan | 2.80 | 3.64 |
| 50 mmol/l NaCitrat/HCl, pH 6<br>50 mmol/l 1,6-Diaminohexan | 3.42 | 4.45 |
| 50 mmol/l NaCitrat/HCl, pH 6<br>50 mmol/l 1,5-Diaminopentan | 3.52 | 4.40 |
| 50 mmol/l NaCitrat/HCl, pH 6<br>50 mmol/l 1,4-Diaminobutan | 3.96 | 5.15 |
| 50 mmol/l NaCitrat/HCl, pH 6<br>50 mmol/l 1,3-Diaminopropan | 3.33 | 4.23 |

**Patentansprüche**

**Patentansprüche für folgende Vertragsstaaten : AT, BE, CH, DE, DK, FR, GB, IT, LI, LU, NL, SE**

1. Pharmazeutisches Präparat eines nicht glykosylierten t-PA-Derivats welches aus der Kringel 2-Domäne und der Proteasedomäne besteht und mit einer der Aminosäuren 174 - 180 beginnt und mit der Aminosäure 527 endet und außerdem die Aminosäuren -3 (Gly) bis +5 (Ile) ganz oder teilweise enthalten kann (K2P pro) mit einer enzymatischen Aktivität von mindestens 1,4 MU/ml und einem pH-Wert von 4,5 bis 6,5, **dadurch gekennzeichnet,** daß es Citrat und mindestens eine Verbindung aus der aus

   a) Ascorbinsäure,
   b) EDTA,
   c) Aminoverbindungen der Formel

   $$R^1R^2N - R - X$$

   wobei X = $SO_3H$, $CH(NH_2)$-$CO_2H$, $CO_2H$, H, $NH_2$ oder OH ist, R = $C_1$-$C_9$-Alkylen, vorzugsweise $C_4$-$C_7$-Alkylen, $C_3$-$C_6$-Cycloalkylen oder Benzyliden ist und $R^1$ und $R^2$ voneinander unabhängig H oder $C_1$-$C_3$-Alkyl sind,
   d) Guanidin-analoge Verbindungen der Formel

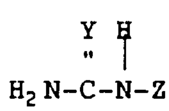

   wobei
   Y = $H_2N^+$ oder 0 ist,
   Z = H oder $(CH_2)_m$ V, $(CH_2)_mCH(NH_2)$-$CO_2H$, $CH(CO_2H)$-$(CH_2)_mCO_2H$ ist,
   V = $NH_2$ oder $CO_2H$ und
   m = 1 bis 4 ist
   e) mit einer oder mehreren Hydroxyl-, Keto- oder/und weiteren Carboxylgruppen substituierten Carbonsäuren,

f) Dimethylbiguanid,
g) Pyrimidinnukleosiden und Pyrimidinnukleotiden,
h) Trehalose, Glucosamin, N-Methylglucamin bestehenden Gruppe enthält.

2. Pharmazeutisches Präparat nach Anspruch 1, **dadurch gekennzeichnet,** daß die Aminoverbindung Taurin, ∊-Aminocapronsäure, Tranexamsäure, Lysin, Ornithin, δ-Aminovaleriansäure, p-Aminomethylbenzoesäure, 4-Aminobutanol-1, 5-Aminopentanol-1, 6-Aminohexanol-1, 1,9-Diaminononan, 1,8-Diaminooctan, 1,7-Diaminoheptan, 1,6-Diaminohexan, 1,5-Diaminopentan, 1,4-Diaminobutan, 1,3-Diaminopropan, 8-Aminooctansäure oder/und 7-Aminoheptansäure ist.

3. Pharmazeutisches Präparat nach Anspruch 1, **dadurch gekennzeichnet,** daß die Guanidin-analoge Verbindung Harnstoff, Guanidinobuttersäure oder/und Arginin ist.

4. Pharmazeutisches Präparat nach Anspruch 1, **dadurch gekennzeichnet,** daß die substituierte Carbonsäure Apfelsäure, Milchsäure, Fumarsäure oder/und 2-Oxoglutarsäure ist.

5. Pharmazeutisches Präparat nach Anspruch 1, **dadurch gekennzeichnet,** daß es zusätzlich eine oder mehrere α-Aminocarbonsäuren, vorzugsweise Histidin enthält.

6. Pharmazeutisches Präparat nach einem der Ansprüche 1 bis 5, **dadurch gekennzeichnet,** daß die Konzentration von Citrat 5 bis 100 mmol/l, vorzugsweise 50 mmol/l beträgt.

7. Pharmazeutisches Präparat nach einem der Ansprüche 1 bis 6, **dadurch gekennzeichnet,** daß es zusätzlich Chlorid-Ionen enthält.

8. Pharmazeutisches Präparat nach Anspruch 1, **dadurch gekennzeichnet,** daß es 50 mmol/l NaCitrat, pH 6, und 0,1 bis 1 mol/l, vorzugsweise 0,2 bis 0,3 mol/l Ascorbinsäure enthält.

9. Pharmazeutisches Präparat nach Anspruch 1, **dadurch gekennzeichnet,** daß es 50 mmol/l NaCitrat, pH 6, und 1 bis 200 mmol/l, vorzugsweise 10 bis 100 mmol/l EDTA enthält.

10. Pharmazeutisches Präparat nach Anspruch 1, **dadurch gekennzeichnet,** daß es 50 mmol/l Nacitrat, pH 6, und 0,1 bis 0,5 mol/l, vorzugsweise 0,1 bis 0,3 mol/l Taurin enthält.

11. Pharmazeutisches Präparat nach Anspruch 1, **dadurch gekennzeichnet,** daß es 50 mmol/l NaCitrat/HCl, pH 6, und 0,5 bis 20 mmol/l, vorzugsweise 1 bis 10 mmol/l ∊-Aminocapronsäure, δ-Aminovaleriansäure, Lysin, Ornithin, Tranexamsäure, p-Aminomethylbenzoesäure, 7-Aminoheptansäure oder 8-Aminooctansäure enthält.

12. Pharmazeutisches Präparat nach Anspruch 1, **dadurch gekennzeichnet,** daß es 50 mmol/l NaCitrat/HCl, pH 6, und 10 bis 100 mmol/l 4-Aminobutanol-1, 5-Aminopentanol-1, 6-Aminohexanol-1, 1,9-Diaminononan, 1,8-Diaminooctan, 1,7-Diaminoheptan, 1,6-Diaminohexan, 1,5-Diaminopentan, 1,4-Diaminobutan oder 1,3-Diaminopropan enthält.

13. Pharmazeutisches Präparat nach Anspruch 1, **dadurch gekennzeichnet,** daß es 50 mmol/l NaCitrat/HCl, pH 6, und 0,1 bis 4 mol/l, vorzugsweise 0,5 bis 2 mol/l Harnstoff enthält.

14. Pharmazeutisches Präparat nach Anspruch 1, **dadurch gekennzeichnet,** daß es 50 mmol/l NaCitrat/HCl, pH 6, 10 bis 200 mmol/l, vorzugsweise 50 bis 100 mmol/l Guanidinobuttersäure oder Arginin enthält.

15. Pharmazeutisches Präparat nach Anspruch 1, **dadurch gekennzeichnet,** daß es 50 mmol/l NaCitrat, pH 6 und 0,001 bis 1 mol/l, vorzugsweise 0,01 bis 0,5 mol/l Apfelsäure, Milchsäure, Fumarsäure oder 2-Oxoglutarsäure enthält.

16. Pharmazeutisches Präparat nach Anspruch 1, **dadurch gekennzeichnet,** daß es 50 mmol/l NaCitrat/HCl, pH 6, und 50 bis 400 mmol/l, vorzugsweise 100 bis 300 mmol/l Dimethylbiguanid enthält.

17. Pharmazeutisches Präparat nach Anspruch 1, **dadurch gekennzeichnet,** daß es 50 mmol/l NaCitrat/HCl, pH 6, 1 bis 300 mmol/l, vorzugsweise 10 bis 300 mmol/l Thymidin, Cytosin oder Uridin enthält.

18. Pharzameutisches Präparat nach Anspruch 1, **dadurch gekennzeichnet,** daß es 50 mmol/l NaCitrat/HCl,

pH 6, und 1 bis 500 mmol/l, vorzugsweise 10 bis 300 mmol/l Trehalose, Glucosamin oder N-Methylglucamin enthält.

**19.** Pharmazeutisches Präparat nach Anspruch 1, **dadurch gekennzeichnet,** daß es 50 mmol/l NaCitrat, pH 6, und eine Kombination von Verbindungen aus der Gruppe a) bis h) nach Anspruch 1 enthält.

**20.** Pharmazeutisches Präparat auf Basis des t-PA-Derivats K2P pro als Wirkstoff, **dadurch gekennzeichnet,** die Zusammensetzung nach einem der vorhergehenden Ansprüche, gegebenenfalls zusammen mit üblichen pharmazeutischen Zusatz-, Hilfs- oder/und Trägerstoffen.

**21.** Verfahren zur Herstellung eines pharmazeutischen Präparats nach einem der Ansprüche 1 bis 20, **dadurch gekennzeichnet,** daß man das t-PA-Derivat K2P pro zusammen mit mindestens einer Substanz aus der Gruppe a) bis h) nach Anspruch 1 in eine geeignete pharmazeutische Darreichungsform überführt.

**22.** Verfahren nach Anspruch 21, **dadurch gekennzeichnet,** daß die pharmazeutische Darreichungsform eine Injektionslösung oder ein Lyophilisat ist.

**23.** Verwendung des t-PA-Derivats K2P pro zur Herstellung von pharmazeutischen Präparaten nach einem der Ansprüche 1 bis 20.

**Patentansprüche für folgende Vertragsstaaten : ES, GR**

**1.** Verfahren zur Herstellung eines pharmazeutischen Präparats eines nicht glykosylierten t-PA-Derivats-welches aus der-Kringel 2-Domäne und der Proteasedomäne besteht und,mit einer der Aminosäuren 174 - 180 beginnt und mit der Aminosäure 527 endet und außerdem die Aminosäuren -3 (Gly) bis +5 (Ile) ganz oder teilweise enthalten kann (K2P pro) mit einer enzymatischen Aktivität von mindestens 1,4 MU/ml und einem pH-Wert von 4,5 bis 6,5, **dadurch gekennzeichnet,** daß man das t-PA-Derivat K2P pro zusammen mit Citrat und mindestens ein er Verbindung aus der aus

a) Ascorbinsäure,
b) EDTA,
c) Aminoverbindungen der Formel

$$R^1R^2N - R - X$$

wobei X = $SO_3H$, $CH(NH_2)$-$CO_2H$, $CO_2H$, H, $NH_2$ oder OH ist, R = $C_1$-$C_9$-Alkylen, vorzugsweise $C_4$-$C_7$-Alkylen, $C_3$-$C_6$-Cycloalkylen oder Benzyliden ist und $R^1$ und $R^2$ voneinander unabhängig H oder $C_1$-$C_3$-Alkyl sind,
d) Guanidin-analoge Verbindungen der Formel

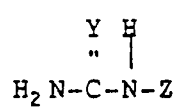

wobei

| | |
|---|---|
| Y | = $H_2N^+$ oder 0 ist, |
| Z | = H oder $(CH_2)_m$ V, $(CH_2)_mCH(NH_2)$-$CO_2H$, $CH(CO_2H)$-$(CH_2)_mCO_2H$ ist, |
| V | = $NH_2$ oder $CO_2H$ und |
| m | = 1 bis 4 ist |

e) mit einer oder mehreren Hydroxyl-, Keto- oder/und weiteren Carboxylgruppen substituierten Carbonsäuren,
f) Dimethylbiguanid,
g) Pyrimidinnukleosiden und Pyrimidinnukleotiden,
h) Trehalose, Glucosamin, N-Methylglucamin bestehenden Gruppein eine geeignete pharmazeutische Darreichungsform überführt.

**2.** Verfahren nach Anspruch 1, **dadurch gekennzeichnet,** daß die Aminoverbindung Taurin, ∊-Aminocapronsäure, Tranexamsäure, Lysin, Ornithin, δ-Aminovaleriansäure, p-Aminomethylbenzoesäure, 4-Aminobutanol-1, 5-Aminopentanol-1, 6-Aminohexanol-1, 1,9-Diaminononan, 1,8-Diaminooctan, 1,7-Diaminoheptan, 1,6-Diaminohexan, 1,5-Diaminopentan, 1,4-Diaminobutan, 1,3-Diaminopropan, 8-Aminooctansäure oder/und 7-Aminoheptansäure ist.

3. Verfahren nach Anspruch 1, **dadurch gekennzeichnet,** daß die Guanidin-analoge Verbindung Harnstoff, Guanidinobuttersäure oder/und Arginin ist.

4. Verfahren nach Anspruch 1, **dadurch gekennzeichnet,** daß die substituierte Carbonsäure Apfelsäure, Milchsäure, Fumarsäure oder/und 2-oxoglutarsäure ist.

5. Verfahren nach Anspruch 1, **dadurch gekennzeichnet,** daß man zusätzlich eine oder mehrere $\alpha$-Aminocarbonsäuren, vorzugsweise Histidin hinzufügt.

6. Verfahren nach einem der Ansprüche 1 bis 5, **dadurch gekennzeichnet,** daß die Konzentration von Citrat 5 bis 100 mmol/l, vorzugsweise 50 mmol/l beträgt.

7. Verfahren nach einem der Ansprüche 1 bis 6, **dadurch gekennzeichnet,** daß man zusätzlich Chlorid-Ionen hinzufügt.

8. Verfahren nach Anspruch 1, **dadurch gekennzeichnet,** daß man 50 mmol/l NaCitrat, pH 6, und 0,1 bis 1 mol/l, vorzugsweise 0,2 bis 0,3 mol/l Ascorbinsäure verwendet.

9. Verfahren nach Anspruch 1, **dadurch gekennzeichnet,** daß man 50 mmol/l NaCitrat, pH 6, und 1 bis 200 mmol/l, vorzugsweise 10 bis 100 mmol/l EDTA verwendet.

10. Verfahren nach Anspruch 1, **dadurch gekennzeichnet,** daß man 50 mmol/l NaCitrat, pH 6, und 0,1 bis 0,5 mol/l, vorzugsweise 0,1 bis 0,3 mol/l Taurin verwendet.

11. Verfahren nach Anspruch 1, **dadurch gekennzeichnet,** daß man 50 mmol/l NaCitrat/HCl, pH 6, und 0,5 bis 20 mmol/l, vorzugsweise 1 bis 10 mmol/l $\epsilon$-Aminocapronsäure, $\delta$-Aminovaleriansäure, Lysin Ornithin, Tranexamsäure, p-Aminomethylbenzoesäure 7-Aminoheptansäure oder 8-Aminooctansäure verwendet.

12. Verfahren nach Anspruch 1, **dadurch gekennzeichnet,** daß man 50 mmol/l NaCitrat/HCl, pH 6, und 10 bis 100 mmol/l 4-Aminobutanol-1, 5-Aminopentanol-1, 6-Aminohexanol-1, 1,9-Diaminononan, 1,8-Diaminooctan, 1,7-Diaminoheptan, 1,6-Diaminohexan, 1,5-Diaminopentan, 1,4-Diaminobutan oder 1,3-Diaminopropan verwendet.

13. Verfahren nach Anspruch 1, **dadurch gekennzeichnet,** daß man 50 mmol/l NaCitrat/HCl, pH 6, und 0,1 bis 4 mol/l, vorzugsweise 0,5 bis 2 mol/l Harnstoff verwendet.

14. Verfahren nach Anspruch 1, **dadurch gekennzeichnet,** daß man 50 mmol/l NaCitrat/HCl, pH 6, 10 bis 200 mmol/l, vorzugsweise 50 bis 100 mmol/l Guanidinobuttersäure oder Arginin verwendet.

15. Verfahren nach Anspruch 1, **dadurch gekennzeichnet,** daß man 50 mmol/l NaCitrat, pH 6 und 0,001 bis 1 mol/l, vorzugsweise 0,01 bis 0,5 mol/l Apfelsäure, Milchsäure, Fumarsäure oder 2-Oxoglutarsäure verwendet.

16. Verfahren nach Anspruch 1, **dadurch gekennzeichnet,** daß man 50 mmol/l NaCitrat/HCl, pH 6, und 50 bis 400 mmol/l, vorzugsweise 100 bis 300 mmol/l Dimethylbiguanid verwendet.

17. Verfahren nach Anspruch 1, **dadurch gekennzeichnet,** daß man 50 mmol/l NaCitrat/HCl, pH 6, 1 bis 300 mmol/l, vorzugsweise 10 bis 300 mmol/l Thymidin, Cytosin oder Uridin verwendet.

18. Verfahren nach Anspruch 1, **dadurch gekennzeichnet,** daß man 50 mmol/l NaCitrat/HCl, pH 6, und 1 bis 500 mmol/l, vorzugsweise 10 bis 300 mmol/l Trehalose, Glucosamin oder N-Methylglucamin verwendet.

19. Verfahren nach Anspruch 1, **dadurch gekennzeichnet,** daß man 50 mmol/l NaCitrat, pH 6, und eine Kombination von Verbindungen aus der Gruppe a) bis h) nach Anspruch 1 verwendet.

20. Verfahren nach einem der vorhergehenden Ansprüche, **dadurch gekennzeichnet,** daß man außerdem übliche pharmazeutische Zusatz-, Hilfs- oder/und Trägerstoffe zusetzt.

**21.** Verfahren nach Anspruch 1, **dadurch gekennzeichnet,** daß die pharmazeutische Darreichungsform eine Injektionslösung oder ein Lyophilisat ist.

**22.** Verwendung des t-PA-Derivats K2P pro zur Herstellung von pharmazeutischen Präparaten nach einem der Ansprüche 1 bis 21.

## Claims

### Claims for the following Contracting States : AT, BE, CH, DE, DK, FR, GB, IT, LI, LU, NL, SE

**1.** Pharmaceutical preparation of a non-glycosylated t-PA derivative which consists of the kringle 2 domain and of the protease domain and begins with one of the amino acids 174 - 180 and ends with the amino acid 527 and, furthermore, can contain the amino acids -3(Gly) to +5(Ile) wholly or partly (K2P pro) with an enzymatic activity of at least 1.4 MU/ml and a pH value of 4.5 to 6.5, characterised in that it contains citrate and at least one compound from the group consisting of

a) ascorbic acid,
b) EDTA,
c) amino compounds of the formula

$$R^1R^2N\text{-}R\text{-}X$$

whereby X = $SO_3H$, $CH(NH_2)\text{-}CO_2H$, $CO_2H$, H, $NH_2$ or OH, R = $C_1$-$C_9$-alkylene, preferably $C_4$-$C_7$-alkylene, $C_3$-$C_6$-cycloalkylene or benzylidene, and $R^1$ and $R^2$, independently of one another, are H or $C_1$-$C_3$-alkyl,
d) guanidine analogous compounds of the formula

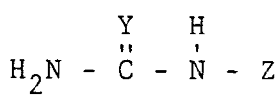

whereby Y = $H_2N^+$ or 0, Z = H or $(CH_2)_mV$, $(CH_2)_mCH(NH_2)\text{-}CO_2H$, $CH(CO_2H)\text{-}(CH_2)_mCO_2H$, V = $NH_2$ or $CO_2H$ and m = 1 to 4,
e) carboxylic acids substituted with one or more hydroxyl, keto and/or further carboxyl groups,
f) dimethylbiguanide,
g) pyrimidine nucleosides and pyrimidine nucleotides,
h) trehalose, glucosamine, N-methylglucamine.

**2.** Pharmaceutical preparation according to claim 1, characterised in that the amino compound is taurine, ε-aminocaproic acid, tranexamic acid, lysine, ornithine, δ-aminovaleric acid, p-aminomethylbenzoic acid, 4-aminobutanol-1, 5-aminopentanol-1, 6-aminohexanol-1, 1,9-diaminononane, 1,8-diaminooctane, 1,7-diaminoheptane, 1,6-diaminohexane, 1,5-diaminopentane, 1,4-diaminobutane, 1,3-diaminopropane, 8-aminooctanoic acid and/or 7-aminoheptanoic acid.

**3.** Pharmaceutical preparation according to claim 1, characterised in that the guanidine-analogous compound is urea, guanidinobutyric acid and/or arginine.

**4.** Pharmaceutical preparation according to claim 1, characterised in that the substituted carboxylic acid is malic acid, lactic acid, fumaric acid and/or 2-oxoglutaric acid.

**5.** Pharmaceutical preparation according to claim 1, characterised in that it additionally contains one or more α-aminocarboxylic acids, preferably histidine.

**6.** Pharmaceutical preparation according to one of claims 1 to 5, characterised in that the concentration of citrate amounts to 5 to 100 mmol/l, preferably 50 mmol/l.

**7.** Pharmaceutical preparation according to one of claims 1 to 6, characterised in that it additionally contains chloride ions.

**8.** Pharmaceutical preparation according to claim 1, characterised in that it contains 50 mmol/l Na citrate,

pH 6, and 0.1 to 1 mol/l, preferably 0.2 to 0.3 mol/ of ascorbic acid.

9. Pharmaceutical preparation according to claim 1, characterised in that it contains 50 mmol/l of Na citrate, pH 6, and 1 to 200 mmol/l, preferably 10 to 100 mmol/l of EDTA.

10. Pharmaceutical preparation according to claim 1, characterised in that it contains 50 mmol/l of Na citrate, pH 6, and 0.1 to 0.5 mol/l, preferably 0.1 to 0.3 mol/l of taurine.

11. Pharmaceutical preparation according to claim 1, characterised in that it contains 50 mmol/l of Na citrate, pH 6, and 0.5 to 20 mmol/l, preferably 1 to 10 mmol/l of $\epsilon$-aminocaproic acid, $\delta$-aminovaleric acid, lysine, ornithine, tranexamic acid, p-aminomethylbenzoic acid, 7-aminoheptanoic acid or 8-aminooctanoic acid.

12. Pharmaceutical preparation according to claim 1, characterised in that it contains 50 mmol/l of Na citrate/HCl, pH 6, and 10 to 100 mmol/l of 4-aminobutanol-1, 5-aminopentanol-1, 6-aminohexanol-1, 1,9-diaminononane, 1,8-diaminooctane, 1,7-diaminoheptane, 1,6-diaminohexane, 1,5-diaminopentane, 1,4-diaminobutane or 1,3-diaminopropane.

13. Pharmaceutical preparation according to claim 1, characterised in that it contains 50 mmol/l of Na citrate/HCl, pH 6, and 0.1 to 4 mol/l, preferably 0.5 to 2 mol/l of urea.

14. Pharmaceutical preparation according to claim 1, characterised in that it contains 50 mmol/l of Na citrate/HCl, pH 6, 10 to 200 mmol/l, preferably 50 to 100 mmol/l of guanidinobutyric acid or arginine.

15. Pharmaceutical preparation according to claim 1, characterised in that it contains 50 mmol/l of Na citrate, pH 6, and 0.001 to 1 mol/l, preferably 0.01 to 5 mol/l of malic acid, lactic acid, fumaric acid or 2-oxoglutaric acid.

16. Pharmaceutical preparation according to claim 1, characterised in that it contains 50 mmol/l of Na citrate/HCl, pH 6, and 50 to 400 mmol/l, preferably 100 to 300 mmol/l of dimethylbiguanide.

17. Pharmaceutical preparation according to claim 1, characterised in that it contains 50 mmol/l of Na citrate/HCl, pH 6, 1 to 300 mmol/l, preferably 10 to 300 mmol/l of thymidine, cytosine or uridine.

18. Pharmaceutical preparation according to claim 1, characterised in that it contains 50 mmol/l of Na citrate/.HCl, pH 6, and 1 to 500 mmol/l, preferably 10 to 300 mmol/l of trehalose, glucosamine or N-methylglucamine.

19. Pharmaceutical preparation according to claim 1, characterised in that it contains 50 mmol/l of Na citrate, pH 6, and a combination of compounds from the group a) to h) according to claim 1.

20. Pharmaceutical preparation based on the t-PA derivative K2P pro as active material, characterised by the composition according to one of the preceding claims, possibly together with usual pharmaceutical additive, adjuvant and/or carrier materials.

21. Process for the preparation of a pharmaceutical preparation according to one of claims 1 to 20, characterised in that one converts the t-PA derivative K2P pro, together with at least one substance from the groups a) to h) according to claim 1, into a suitable pharmaceutical form of administration.

22. Process according to claim 21, characterised in that the pharmaceutical form of administration is an injection solution or a lyophilisate.

23. Use of the t-PA derivative K2P pro for the production of pharmaceutical preparations according to one of claims 1 to 20.

**Claims for the following Contracting States : ES, GR**

1. Process for the production of a pharmaceutical preparation of a non-glycosylated t-PA derivative which consists of the kringle 2 domain and of the protease domain and begins with one of the amino acids 174 - 180 and ends with the amino acid 527 and, furthermore, can contain the amino acids -3(Gly) to +5(Ile) wholly or partly (K2P pro) with an enzymatic activity of at least 1.4 MU/ml and a pH value of 4.5 to 6.5,

characterised in that one converts the t-PA derivative, together with citrate and at least one compound from the group consisting of

a) ascorbic acid,

b) EDTA,

c) amino compounds of the formula

$$R^1R^2N-R-X$$

whereby X = $SO_3H$, $CH(NH_2)-CO_2H$, $CO_2H$, H, $NH_2$ or OH, R = $C_1$-$C_9$-alkylene, preferably $C_4$-$C_7$-alkylene, $C_3$-$C_6$-cycloalkylene or benzylidene and $R^1$ and $R^2$, independently of one another, are H or $C_1$-$C_3$-alkyl,

d) guanidine analogous compounds of the formula

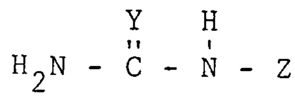

whereby Y = $H_2N^+$ or 0, Z = H or $(CH_2)_mV$, $(CH_2)_mCH(NH_2)-CO_2H$, $CH(CO_2H)-(CH_2)_mCO_2H$, V = $NH_2$ or $CO_2H$ and m = 1 to 4,

e) carboxylic acids substituted with one or more hydroxyl, keto and/or further carboxyl groups,

f) dimethylbiguanide,

g) pyrimidine nucleosides and pyrmidine nucleotides,

h) trehalose, glucosamine, N-methylglucamine, into a suitable pharmaceutical form of administration.

**2.** Process according to claim 1, characterised in that the amino compound is taurine, ∈-aminocaproic acid, tranexamic acid, lysine, ornithine, δ-aminovaleric acid, p-aminomethylbenzoic acid, 4-aminobutanol-1, 5-aminopentanol-1, 6-aminohexanol-1, 1,9-diaminononane, 1,8-diaminooctane, 1,7-diaminoheptane, 1,6-diaminohexane, 1,5-diaminopentane, 1,4-diaminobutane, 1,3-diaminopropane, 8-aminooctanoic acid and/or 7-aminoheptanoic acid.

**3.** Process according to claim 1, characterised in that the guanidine-analogous compound is urea, guanidinobutyric acid and/or arginine.

**4.** Process according to claim 1, characterised in that the substituted carboxylic acid is malic acid, lactic acid, fumaric acid and/or 2-oxoglutaric acid.

**5.** Process according to claim 1, characterised in that one additionally adds thereto one or more α-aminocarboxylic acids, preferably histidine.

**6.** Process according to one of claims 1 to 5, characterised in that the concentration of citrate amounts to 5 to 100 mmol/l, preferably 50 mmol/l.

**7.** Process according to one of claims 1 to 6, characterised in that one additionally adds chloride ions thereto.

**8.** Process according to claim 1, characterised in that one uses 50 mmol/l of Na citrate, pH 6, and 0.1 to 1 mol/l, preferably 0.2 to 0.3 mol/ of ascorbic acid.

**9.** Process according to claim 1, characterised in that one uses 50 mmol/l of Na citrate, pH 6, and 1 to 200 mmol/l, preferably 10 to 100 mmol/l of EDTA.

**10.** Process according to claim 1, characterised in that one uses 50 mmol/l of Na citrate, pH 6, and 0.1 to 0.5 mol/l, preferably 0.1 to 0.3 mol/l of taurine.

**11.** Process according to claim 1, characterised in that one uses 50 mmol/l of Na citrate, pH 6, and 0.5 to 20 mmol/l, preferably 1 to 10 mmol/l of ∈-aminocaproic acid, δ-aminovaleric acid, lysine, ornithine, tranexamic acid, p-aminomethylbenzoic acid, 7-aminoheptanoic acid or 8-aminooctanoic acid.

**12.** Process according to claim 1, characterised in that one uses 50 mmol/l of Na citrate/HCl, pH 6, and 10 to 100 mmol/l of 4-aminobutanol-1, 5-aminopentanol-1, 6-aminohexanol-1, 1,9-diaminononane, 1,8-diaminooctane, 1,7-diaminoheptane, 1,6-diaminohexane, 1,5-diaminopentane, 1,4-diaminobutane or 1,3-diaminopropane.

**13.** Process according to claim 1, characterised in that one uses 50 mmol/l of Na citrate/HCl, pH 6, and 0.1 to 4 mol/l, preferably 0.5 to 2 mol/l of urea.

**14.** Process according to claim 1, characterised in that one uses 50 mmol/l of Na citrate/HCl, pH 6, 10 to 200 mmol/l, preferably 50 to 100 mmol/l of guanidinobutric acid or arginine.

**15.** Process according to claim 1, characterised in that one uses 50 mmol/l of Na citrate, pH 6, and 0.001 to 1 mol/l, preferably 0.01 to 5 mol/l of malic acid, lactic acid, fumaric acid or 2-oxoglutaric acid.

**16.** Process according to claim 1, characterised in that one uses 50 mmol/l of Na citrate/HCl, pH 6, and 50 to 400 mmol/l, preferably 100 to 300 mmol/l of dimethylbiguanide.

**17.** Process according to claim 1, characterised in that one uses 50 mmol/l of Na citrate/HCl, pH 6, 1 to 300 mmol/l, preferably 10 to 300 mmol/l of thymidine, cytosine or uridine.

**18.** Process according to claim 1, characterised in that one uses 50 mmol/l of Na citrate/HCl, pH 6, and 1 to 500 mmol/l, preferably 10 to 300 mmol/l of trehalose, glucosamine or N-methylglucamine.

**19.** Process according to claim 1, characterised in that one uses 50 mmol/l of Na citrate, pH 6, and a combination of compounds from the group a) to h) according to claim 1.

**20.** Process according to one of the preceding claims, characterised in that one also adds usual pharmaceutical additive, adjuvant and/or carrier materials.

**21.** Process according to claim 1, characterised in that the pharmaceutical form of administration is an injection solution or a lyophilisate.

**22.** Use of the t-PA derivative K2P pro for the production of pharmaceutical preparations according to one of claims 1 to 21.

## Revendications

### Revendications pour les Etats Contractants suivants : AT, BE, CH, DE, DK, FR, GB, IT, LI, LU, NL, SE

**1.** Préparation pharmaceutique d'un dérivé non glycosylé du t-PA qui consiste en le domine de boucle 2 et en le domaine de protéase, qui commence par l'un des acides aminés 174 - 180 et qui se termine par l'acide aminé 527, et qui peut contenir en outre totalement ou partiellement les acides aminés - 3 (Gly) à + 5 (Ile), (K2P pro), présentant une activité enzymatique d'au moins 1,4 MU/ml et un pH de 4,5 à 6,5, caractérisée en ce qu'elle contient du citrate et au moins un composé du groupe formé par

a) l'acide ascorbique,

b) l'EDTA

c) un composé aminé de formule

$$R^1R^2N - R - X$$

dans laquelle X = $SO_3H$, $CH(NH_2)$-$CO_2H$, $CO_2H$, H, $NH_2$ ou OH, R = alkylène en $C_1$ - $C_9$, de préférence alkylène en $C_4$ - $C_7$, cycloalkylène en $C_3$ - $C_6$ ou benzylidène et $R^1$ et $R^2$ sont indépendamment l'un de l'autre H ou alkyle en $C_1$ - $C_3$,

d) des composés analogues à la guanidine de formule

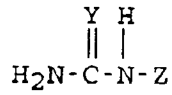

dans laquelle Y = $H_2N^+$ ou O,

Z = H ou $(CH_2)_mV$, $(CH_2)_mCH(NH_2)$-$CO_2H$, $CH(CO_2H)$-$(CH_2)_mCO_2H$, V = $NH_2$ ou $CO_2H$ et

m = 1 à 4

e) des acides carboxyliques substitués par un ou plusieurs groupes hydroxyle, cétone et/ou groupes

carboxyliques supplémentaires,
f) le diméthylbiguanide,
g) des nucléosides pyrimidiques et des nucléotides pyrimidiques,
h) le tréhalose, la glucosamine, la N-méthylglucamine.

2. Préparation pharmaceutique selon la revendication 1, caractérisée en ce que le composé aminé est la taurine, l'acide ∊-aminocaproïque, l'acide tranexamique, la lysine, l'ornithine, l'acide δ-aminovalérique, l'acide p-aminométhylbenzoïque, le 4-aminobutanol-1, le 5-aminopentanol-1, le 6-aminohexanol-1, le 1,9-diaminononane, le 1,8-diaminooctane, le 1,7-diaminoheptane, le 1,6-diaminohexane, le 1,5-diaminopentane, le 1,4-diaminobutane, le 1,3-diaminopropane, l'acide 8-aminooctanoïque et/ou l'acide 7-aminoheptanoïque.

3. Préparation pharmaceutique selon la revendication 1, caractérisée en ce que le composé analogue à la guanidine est l'urée, l'acide guanidinobutyrique et/ou l'arginine.

4. Préparation pharmaceutique selon la revendication 1, caractérisée en ce que l'acide carboxylique substitué est l'acide malique, l'acide lactique, l'acide fumarique et/ou l'acide 2-oxoglutarique.

5. Préparation pharmaceutique selon la revendication 1, caractérisée en ce qu'elle contient en outre un ou plusieurs acides α-aminocarboxyliques, de préférence l'histidine.

6. Préparation pharmaceutique selon l'une des revendications 1 à 5, caractérisée en ce que la concentration du citrate est de 5 à 100 mmol/l, de préférence de 50 mmol/l.

7. Préparation pharmaceutique selon l'une des revendications 1 à 6, caractérisée en ce qu'elle contient en outre des ions chlorure.

8. Préparation pharmaceutique selon la revendication 1, caractérisée en ce qu'elle contient 50 mmol/l de citrate de Na, pH 6, et 0,1 à 1 mol/l, de préférence 0,2 à 0,3 mol/l d'acide ascorbique.

9. Préparation pharmaceutique selon la revendication 1, caractérisée en ce qu'elle contient 50 mmol/l de citrate de Na, pH 6, et 1 à 200 mmol/l, de préférence 10 à 100 mmol/l de EDTA.

10. Préparation pharmaceutique selon la revendication 1, caractérisée en ce qu'elle contient 50 mmol/l de citrate de Na, pH 6, et 0,1 à 0,5 mol/l, de préférence 0,1 à 0,3 mol/l de taurine.

11. Préparation pharmaceutique selon la revendication 1, caractérisée en ce qu'elle contient 50 mmol/l de citrate de Na/HCl, pH 6, et 0,5 à 20 mmol/l, de préférence 1 à 10 mmol/l d'acide ∊-aminocaproïque, d'acide δ-aminovalérique, de lysine, d'ornithine, d'acide tranexamique, d'acide p-aminométhylbenzoïque, d'acide 7-aminoheptanoïque ou d'acide 8-aminooctanoïque.

12. Préparation pharmaceutique selon la revendication 1, caractérisée en ce qu'elle contient 50 mmol/l de citrate de Na/HCl, pH 6, et 10 à 100 mmol/l de 4-aminobutanol-1, de 5-aminopentanol-1, de 6-aminohexanol-1, de 1,9-diaminononane, de 1,8-diaminooctane, de 1,7-diaminoheptane, de 1,6-diaminohexane, de 1,5-diaminopentane, de 1,4-diaminobutane ou de 1,3-diaminopropane.

13. Préparation pharmaceutique selon la revendication 1, caractérisée en ce qu'elle contient 50 mmol/l de citrate de Na/HCl, pH 6, et 0,1 à 4 mol/l, de préférence 0,5 à 2 mol/l d'urée.

14. Préparation pharmaceutique selon la revendication 1, caractérisée en ce qu'elle contient 50 mmol/l de citrate de Na/HCl, pH 6, 10 à 200 mmol/l, de préférence 50 à 100 mmol/l d'acide guanidinobutyrique ou d'arginine.

15. Préparation pharmaceutique selon la revendication 1, caractérisée en ce qu'elle contient 50 mmol/l de citrate de Na, pH 6, et 0,001 à 1 mol/l, de préférence 0,01 à 0,5 mol/l d'acide malique, d'acide lactique, d'acide fumarique ou d'acide 2-oxoglutarique.

16. Préparation pharmaceutique selon la revendication 1, caractérisée en ce qu'elle contient 50 mmol/l de citrate de Na/HCl, pH 6, et 50 à 400 mmol/l, de préférence 100 à 300 mmol/l de diméthylbiguanide.

**17.** Préparation pharmaceutique selon la revendication 1, caractérisée en ce qu'elle contient 50 mmol/l de citrate de Na/HCl, pH 6, 1 à 300 mmol/l, de préférence 10 à 300 mmol/l de thymidine, de cytosine ou d'uridine.

**18.** Préparation pharmaceutique selon la revendication 1, caractérisée en ce qu'elle contient 50 mmol/l de citrate de Na/HCl, pH 6, et 1 à 500 mmol/l, de préférence 10 à 300 mmol/l de tréhalose, de glucosamine ou de N-méthylglucamine.

**19.** Préparation pharmaceutique selon la revendication 1, caractérisée en ce qu'elle contient 50 mmol/l de citrate de Na, pH 6, et une combinaison de composés du groupe a) à h) selon la revendication 1.

**20.** Préparation pharmaceutique à base de dérivé K2P pro du t-PA en tant que principe actif, caractérisée par la composition selon l'une des revendications précédentes, éventuellement avec des additifs, adjuvants et/ou véhicules pharmaceutiques habituels.

**21.** Procédé d'obtention d'une préparation pharmaceutique selon l'une des revendications 1 à 20, caractérisé en ce que l'on convertit en une forme d'administration pharmaceutique appropriée le dérivé K2P pro du t-PA et au moins une substance du groupe a) à h) selon la revendication 1.

**22.** Procédé selon la revendication 21, caractérisé en ce que la forme d'administration pharmaceutique est une solution pour injection ou un lyophilisat.

**23.** Utilisation du dérivé K2P pro du t-PA pour l'obtention de préparations pharmaceutiques selon l'une des revendications 1 à 20.

**Revendications pour les Etats Contractants suivants : ES, GR**

**1.** Procédé d'obtention d'une préparation pharmaceutique d'un dérivé non glycosylé du t-PA qui consiste en le domaine de boucle 2 et en le domaine de protéase, qui commence par l'un des acides aminés 174 - 180 et qui se termine par l'acide aminé 527, et qui peut contenir en outre totalement ou partiellement les acides aminés - 3 (Gly) à + 5 (Ile), (K2P pro), présentant une activité enzymatique d'au moins 1,4 MU/ml et un pH de 4,5 à 6,5, caractérisé en ce que l'on convertit en une forme d'administration pharmaceutique appropriée le dérivé K2P pro du t-PA avec du citrate et au moins un composé du groupe formé par

a) l'acide ascorbique,
b) l'EDTA
c) un composé aminé de formule

$$R^1R^2N - R - X$$

dans laquelle X = $SO_3H$, $CH(NH_2)$-$CO_2H$, $CO_2H$, H, $NH_2$ ou OH, R = alkylène en $C_1$ - $C_9$, de préférence alkylène en $C_4$ - $C_7$, cycloalkylène en $C_3$ - $C_6$ ou benzylidène et $R^1$ et $R^2$ sont indépendamment l'un de l'autre H ou alkyle en $C_1$ - $C_3$,
d) des composés analogues à la guanidine de formule

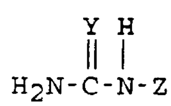

dans laquelle Y = $H_2N^+$ ou O,
Z = H ou $(CH_2)_mV$, $(CH_2)_mCH(NH_2)$ -$CO_2H$, $CH(CO_2H)$ - $(CH_2)_mCO_2H$, V = $NH_2$ ou $CO_2H$ et m=1 à 4
e) des acides carboxyliques substitués par un ou plusieurs groupes hydroxyle, cétone et/ou groupes carboxyliques supplémentaires,
f) le diméthylbiguanide,
g) des nucléosides pyrimidiques et des nucléotides pyrimidiques,
h) le tréhalose, la glucosamine, la N-méthylglucamine.

**2.** Procédé selon la revendication 1, caractérisé en ce que le composé aminé est la taurine, l'acide ∊-aminocaproïque, l'acide tranexamique, la lysine, l'ornithine, l'acide δ-aminovalérique, l'acide p-aminométhylbenzoïque, le 4-aminobutanol-1, le 5-aminopentanol-1, le 6-aminohexanol-1, le 1,9-diaminononane, le

1,8-diaminooctane, le 1,7-diaminoheptane, le 1,6-diaminohexane, le 1,5-diaminopentane, le 1,4-diaminobutane, le 1,3-diaminopropane, l'acide 8-aminooctanoïque et/ou l'acide 7-aminoheptanoïque.

3. Procédé selon la revendication 1, caractérisé en ce que le composé analogue à la guanidine est l'urée, l'acide guanidinobutyrique et/ou l'arginine.

4. Procédé selon la revendication 1, caractérisé en ce que l'acide carboxylique substitué est l'acide malique, l'acide lactique, l'acide fumarique et/ou l'acide 2-oxoglutarique.

5. Procédé selon la revendication 1, caractérisé en ce que l'on ajoute en outre un ou plusieurs acides α-aminocarboxyliques; de préférence l'histidine.

6. Procédé selon l'une des revendications 1 à 5, caractérisé en ce que la concentration du citrate est de 5 à 100 mmol/l, de préférence de 50 mmol/l.

7. Procédé selon l'une des revendications 1 à 6, caractérisé en ce que l'on ajoute en outre des ions chlorure.

8. Procédé selon la revendication 1, caractérisé en ce que l'on utilise 50 mmol/l de citrate de Na, pH 6, et 0,1 à 1 mol/l, de préférence 0,2 à 0,3 mol/l d'acide ascorbique.

9. Procédé selon la revendication 1, caractérisé en ce que l'on utilise 50 mmol/l de citrate de Na, pH 6, et 1 à 200 mmol/l, de préférence 10 à 100 mmol/l de EDTA.

10. Procédé selon la revendication 1, caractérisé en ce que l'on utilise 50 mmol/l de citrate de Na, pH 6, et 0,1 à 0,5 mol/l, de préférence 0,1 à 0,3 mol/l de taurine.

11. Procédé selon la revendication 1, caractérisé en ce que l'on utilise 50 mmol/l de citrate de Na/HCl, pH 6, et 0,5 à 20 mmol/l, de préférence 1 à 10 mmol/l d'acide ∊-aminocaproïque, d'acide δ-aminovalérique, de lysine, d'ornithine, d'acide tranexamique, d'acide p-aminométhylbenzoïque, d'acide 7-aminoheptanoïque ou d'acide 8-aminooctanoïque.

12. Procédé selon la revendication 1, caractérisé en ce que l'on utilise 50 mmol/l de citrate de Na/HCl, pH 6, et 10 à 100 mmol/l de 4-aminobutanol-1, de 5-aminopentanol-1, de 6-aminohexanol-1, de 1,9-diaminononane, de 1,8-diaminooctane, de 1,7-diaminoheptane, de 1,6-diaminohexane, de 1,5-diaminopentane, de 1,4-diaminobutane ou de 1,3-diaminopropane.

13. Procédé selon la revendication 1, caractérisé en ce que l'on utilise 50 mmol/l de citrate de Na/HCl, pH 6, et 0,1 à 4 mol/l, de préférence 0,5 à 2 mol/l d'urée.

14. Procédé selon la revendication 1, caractérisé en ce que l'on utilise 50 mmol/l de citrate de Na/HCl, pH 6, 10 à 200 mmol/l, de préférence 50 à 100 mmol/l d'acide guanidinobutyrique ou d'arginine.

15. Procédé selon la revendication 1, caractérisé en ce que l'on utilise 50 mmol/l de citrate de Na, pH 6, et 0,001 à 1 mol/l, de préférence 0,01 à 0,5 mol/l d'acide malique, d'acide lactique, d'acide fumarique ou d'acide 2-oxoglutarique.

16. Procédé selon la revendication 1, caractérisé en ce que l'on utilise 50 mmol/l de citrate de Na/HCl, pH 6, et 50 à 400 mmol/l, de préférence 100 à 300 mmol/l de diméthylbiguanide.

17. Procédé selon la revendication 1, caractérisé en ce que l'on utilise 50 mmol/l de citrate de Na/HCl, pH 6, 1 à 300 mmol/l, de préférence 10 à 300 mmol/l de thymidine, de cytosine ou d'uridine.

18. Procédé selon la revendication 1, caractérisé en ce que l'on utilise 50 mmol/l de citrate de Na/HCl, pH 6, et 1 à 500 mmol/l, de préférence 10 à 300 mmol/l de tréhalose, de glucosamine ou de N-méthylglucamine.

19. Procédé selon la revendication 1, caractérisé en ce que l'on utilise 50 mmol/l de citrate de Na, pH 6, et une combinaison de composés du groupe a) à h) selon la revendication 1.

20. Procédé selon l'une des revendications précédentes, caractérisé en ce que l'on ajoute en outre des additifs, adjuvants et/ou véhicules pharmaceutiques habituels.

**21.** Procédé selon la revendication 1, caractérisé en ce que la forme d'administration pharmaceutique est une solution pour injection ou un lyophilisat.

**22.** Utilisation du dérivé K2P pro du t-PA pour l'obtention de préparations pharmaceutiques selon l'une des revendications 1 à 21.